# EUROPÄISCHE PATENTSCHRIFT

(11) **EP 0 642 020 B1**
(45) Veröffentlichungstag und Bekanntmachung des Hinweises auf die Patenterteilung: **15.04.1998**
(21) Anmeldenummer: 93912021.8
(22) Anmeldetag: 06.05.1993
(51) Int. Cl.: G01N 33/493, G01N 25/14, G01N 33/49, G01N 33/68

(54) **VERFAHREN ZUR FESTSTELLUNG DER STEINBILDUNGSAKTIVITÄT UND DES GEHALTES AN STEINBILDENDEN SALZEN VON URIN BEI UROLITHIASE**
METHOD FOR DETERMINING LITHOGENESIS ACTIVITY RATE AND URINA LITHOGENETIC SALTS CONTENT IN UROLITHIASIS
PROCEDE DE DETERMINATION DU TAUX D'ACTIVITE DE LITHOGENESE ET DE LA TENEUR EN SELS LITHOGENES DE L'URINE EN UROLITHIASE

(30) Priorität: 21.05.1992 RU 5994814; 30.06.1992 RU 6543214
(43) Veröffentlichungstag der Anmeldung: 08.03.1995
(73) Patentinhaber: SHABALIN, Vladimir Nikolaevich, Moscow, 129301 (SU); SHATOKHINA, Svetlana Nikolaevna, Moscow, 129041 (SU)
(72) Erfinder: SHABALIN, Vladimir Nikolaevich, Moscow, 129301 (SU); SHATOKHINA, Svetlana Nikolaevna, Moscow, 129041 (SU)
(74) Vertreter: Nix, Frank Arnold, Dr.
(86) Internationale Anmeldenummer: RU9300105
(87) Internationale Veröffentlichungsnummer: WO9323753

(56) Entgegenhaltungen:
- EP-A- 0 252 762
- EP-A- 0 292 311
- EP-A- 0 307 566
- WO-A-92/02821
- DE-A- 3 643 263
- US-A- 4 183 729

## Beschreibung

### Gebiet der Technik

Die Erfindung betrifft ein Analysenverfahren auf dem Gebiet der Medizin, insbesondere ein Verfahren zur Bestimmung der Steinbildungsaktivität und der Zusammensetzung steinbildender Urinsalze bei einer Urolithiasis.

### Stand der Technik

Derzeit sind die Probleme der dynamischen Untersuchung des Steinbildungsvorganges im menschlichen Organismus, dessen Intensität, der Ermittlung der Salzzusammensetzung im Zuge der Steinbildung aktuell, weil sie es ermöglichen, einer Urinsteinbildung vorzubeugen, eine Korrektur bei dem genannten Vorgang rechtzeitig vorzunehmen, eine individuelle Therapie bei der Urolithiasis festzulegen und zu realisieren.

Bekannt sind bereits Verfahren zur Vorhersage von Urolithiasis, die auf der Feststellung eines Steinbildungsvorganges beruhen, wie z.B. ein Verfahren zur Vorhersage durch die Entdeckung von Urinkristallen in frisch abgeschiedenem Urin oder im Urin kurz nach dem Urinieren (W.Je.Predtetschenski. "Anleitung zu klinischen Laboruntersuchungen", 1964, Verl. "Medizina", S. 420 bis 446), oder ein Verfahren zur Vorhersage von Urolithiasis (SU, A, 16 29 846), das sich auf der Bestimmung der kristallbildenden Urinaktivität durch das Vermengen von Kalziumchlorid- und Natriumoxalat-Salzlösungen mit Urin, die Exposition der Mischung und die Zählung der gebildeten Kristalle aufbaut.

Jedoch sind diese Verfahren lediglich auf die Entdeckung eines Steinbildungsvorganges ausgerichtet und ermöglichen es nicht, den Aktivitätsgrad dieses Prozesses zu beurteilen.

Man kennt ferner ein Verfahren zur Urolithiasisvorhersage, bei dem vorgeschlagen ist, das Gefährdungspotential der Kalziumphosphatkristallisation im Urin durch die Zählung der gebildeten Kristalle einer bestimmten Größe unter Verwendung mathematischer Berechnungen zu bestimmen ("Urological Research, N 2, 15, 1987, Springer-Verlag, H.-G.Tiselius, "Measurement of the Risk of Calcium Phosphate Crystallization in Urine ", p. 79-81). Dieses Verfahren ist durch die mathematischen Berechnungen erschwert und hat ebenfalls nur die Feststellung eines Steinbildungsvorganges zur Aufgabe.

Bekannt ist weiter ein Verfahren zur Urolithiasisvorhersage (WO 92/02821) , bei dem einer Urinprobe eine Eiweißlösung wie z.B. Albumin zugesetzt, die Mischung getrocknet und nach einer vollständigen Kristallisation der Probe die Urolithiasis prognostiziert wird. Dieses Verfahren dient einzig und allein zur Entdeckung eines Steinbildungsvorganges und ist für die Bestimmung des Steinbildungs-Aktivitätsgrades nicht geeignet.

Darüber hinaus sind Verfahren zur Bestimmung der Salzzusammensetzung bei Urolithiasis bekannt (W.Je.Predtetschenski. "Anleitung zu klinischen Laboruntersuchungen", 1964, Verl. "Medizina" Moskau, S.452 bis 454; Wandt M., Underhill L. "Brit. J.Urol.", 1988, 61, N 6, 478-481; Schubert G., Brien G., Adam K. "Z.Klin. Med.", 1989, 44, Nr. 11, S.923 bis 928; Nichino T., Sakura T., Sato T., Koiso K., Kaneko S., "Jap. J. Nephrol", 1987, 29, N 5, 571-575). All diese Verfahren beruhen auf der Untersuchung der Urinkomponenten (des Salzsedimentes, des Sandes, der Konkremente) auf physikalisch-chemischem Wege unter Verwendung der Adsorption, des Ionenaustausches wie z.B. der Chromatografie, mit Hilfe der Röntgenspektralanalyse, der Röntgenstrukturanalyse, der Thermoanalyse mittels magnetischer Kernresonanz, der paramagnetischen Elektronenresonanz oder anderer Spin-Effekte.

Die genannten Verfahren eignen sich nur für die Untersuchung der gebildeten Steine, die sich aus dem Organismus abgeschieden haben, und ermöglichen es nicht, die chemische Natur der Steinbildung in frühen Stufen der Urolithiasis und der gebildeten, jedoch nicht aus dem Organismus abgeschiedenen Steine zu bestimmen.

Ein Verfahren zur Bestimmung der chemischen Zusammensetzung der steinbildenden Salze in frühen Stufen der Urolithiasis und der gebildeten, jedoch nicht aus dem Organismus abgeschiedenen Steine ist nicht bekannt.

### Offenbarung der Erfindung

Das anmeldungsgemäße Verfahren zur Bestimmung des Steinbildungsaktivitätsgrades und der Zusammensetzung der steinbildenden Urinsalze bei einer Urolithiasis ist neu und in der Fachliteratur nicht beschrieben.

Der Erfindung wurde die Aufgabe zugrunde gelegt, ein Verfahren zu schaffen, das es ermöglicht, schnell und genau den Steinbildungsaktivitätsgrad bei Urolithiasis und die Salzzusammensetzung des Steines in beliebiger Stufe dessen Bildung zu bestimmen.

Diese Aufgabe ist dadurch gelöst worden, daß bei einem Verfahren zur Bestimmung des Steinbildungsaktivitätsgrades und der Zusammensetzung steinbildender Urinsalze bei einer Urolithiasis durch Zusatz einer wäßrigen Eiweißlösung zu einer Urinprobe bei einer Aufbringung der erhaltenen Mischung in Form eines Tropfens auf eine ebene Oberfläche, Trocknung innerhalb von 24 Stunden und Ermittlung der Art der Salzkristallisation in der Randzone der Probe gemäß der Erfindung der Urinprobe die Eiweißlösung im Verhältnis von 9:1, 7:1 oder 5:1 zugesetzt wird und bei Vorhandensein einzelner Kristalle, deren Konglomerate oder bei vollständiger Kristallisation dieser Zone über einen schwachen, gemäßigten oder ausgeprägten Steinbildungsaktivitätsgrad geschlußfolgert und die Zusammensetzung dieser kristallinen Bildungen unter paralleler Bestimmung der Salzzusammensetzung in der Mittelzone der Probe und bei späterer Durchführung einer Vergleichsanalyse dieser Zusammensetzungen zwecks Bestimmung der steinbildenden Urinsalze ermittelt wird.

Es ist hierbei vorteilhaft, daß als wäßrige Eiweißlösung eine 8-12%ige wäßrige Albuminlösung zum Einsatz gelangt. Es ist weiter sinnvoll, daß zur Verbesserung des Analysenbefundes der auf die ebene Oberfläche aufgetragene Tropfen der Mischung bei Raumtemperatur getrocknet wird. Um die Analyse zu beschleunigen und deren Ergebnisse zu verbessern, ist es ratsam, zur Bestimmung der Elemente als Bestandteile der erwähnten kristallinen Bildungen eine Röntgenspektralmikroanalyse durchzuführen.

Dank dem erfindungsgemäßen Verfahren wurde der Zusammenhang zwischen der Steinbildungsaktivität und der Salzkristallisationsintensität in der Randzone einer Urinzone festgestellt. Dies ermöglicht es, in einer Urinprobe schnell und genau den Steinbildungsaktivitätsgrad zu bestimmen, so daß dadurch die Untersuchung des Steinbildungsvorganges in der Dynamik durchgeführt, rechtzeitig eine Korrektur bei dem genannten Vorgang vorgenommen, die Bildung eines Urinsteines vermieden und im Bedarfsfall eine individuelle Therapie durchgeführt werden kann. Hinzu kommt, daß das anmeldungsgemäße Verfahren es gestattet, vor der Steinbildung im Organismus die Zusammensetzung steinbildender Salze zu bestimmen und zielgesetzte Maßnahmen zu deren Ausscheidung aus dem Organismus und zur Vorbeuge des Eindringens dieser Salze gemeinsam mit Trinkwasser und Lebensmitteln einzuleiten. Das angemeldete Verfahren bietet die Möglichkeit dafür, daß binnen einer bestimmten Beobachtungszeit bei einem Kranken mit einem in den Harnwegen befindlichen Stein festgestellt wird, aus welchen steinbildenden Salzen die Oberschicht des Steines besteht, was beispielsweise bei der Lithotripsie unerläßlich sein kann.

Die Untersuchung der Zusammensetzung steinbildender Salze in der Dynamik (auch von Anfang an) der Steinbildung bietet die Möglichkeit für die Ermittlung der Zusammensetzung und der späteren Struktur des ganzen Steines, was für die Durchführung der Lithotripsie von Bedeutung ist.

Darüber hinaus gestattet das angemeldete Analysenverfahren, eine zielgerichtete Vorbeugung des Urolithiasisrezidivierens zu realisieren. Das Verfahren ist einfach bei der Ausführung und leicht zu handhaben, es setzt keine besondere gerätetechnische Gestaltung voraus.

### Ausführungsform der Erfindung

Das anmeldungsgemäße Verfahren zur Bestimmung des Steinbildungsaktivitätsgrades beruht, wie eingangs erwähnt, auf dem herausgefundenen Zusammenhang zwischen der Salzkristallisation in der Randzone einer Urinprobe und der Steinbildungsaktivität. Dieses Verfahren läßt sich wie folgt realisieren.

Einer Urinprobe wird eine wäßrige Eiweißlösung (vorteilhaft eine 8-12%ige wäßrige Albuminlösung) in einem Verhältnis von 9:1, 7:1 oder 5:1 zugesetzt. Für die Analyse kann eine Urinprobe mit einer Eiweißlösung in einem der genannten Verhältnisse eingestzt werden. Um die Aussagekräftigkeit der Analyse zu sichern, können zwei oder drei Varianten der genannten Verhältnisse verwendet werden. Anschließend wird ein Tropfen der erhaltenen Mischung auf eine plane Oberfläche aufgebracht und innerhalb von mindestens 24 Stunden bei Raumtemperatur getrocknet.

Beim Trocknen bestimmt man die Kristallisationsintensität der Urinsalze in der Randzone der Probe : Einzelkristalle deuten auf eine schwache Aktivität des Steinbildungsvorganges, vorhandene Konglomerate der Salzkristalle auf eine gemäßigte Aktivität und eine volle Salzkristallisation in dieser Zone auf eine ausgeprägte Steinbildungsaktivität hin.

Das Fehlen von Kristallen in der Randzone ist ein Anzeichen dafür, daß keine Steinbildung geschieht. Das anmeldungsgemäße Verfahren ermöglicht zugleich, den Aktivitätsgrad und die Salzzusammensetzung steinbildender Urinsalze zu erkennen. Bei Vorhandensein von kristallinen Bildungen in der Randzone einer Probe bestimmt man unter Verwendung physikalischer Verfahren (vorzugsweise der Röntgenspektralmikroanalyse) die Elementzusammensetzung der kristallinen Bildungen. Parallel dazu ermittelt man die Salzzusammensetzung in der Mittelzone der Urinprobe und führt eine Vergleichsanalyse dieser Zusammensetzungen durch, auf deren Grundlage dann auf die chemische Zusammensetzung der steinbildenden Salze geschlossen wird.

Steinbildung ist ein dynamischer Vorgang, dessen Salzzusammensetzung sich während der Zeit verändern kann. Das anmeldungsgemässe Verfahren bietet die Möglichkeit für die Bestimmung der Salzzusammensetzung eines Steines, der sich erst bildet oder sich künftig bei der vorliegenden Urinsalzzusammensetzung bilden kann.

Das angemeldete Verfahren wurde in klinischen Laboratorien getestet. Um den Steinbildungsaktivitätsgrad zu ermitteln, wurden 12 steinausscheidende Kranke (also Kranke, bei denen ein spontaner Bruch und eine Ausscheidung von Steinen regelmäßig beobachtet werden) und 128 praktisch gesunde Leute, die sich in vorklinischer Urolithiasisstufe befanden, was durch ein früher bekanntes Verfahren festgestellt wurde, und deren Steinbildungsaktivitätsgrad jedoch nicht bekannt war, untersucht. Die Untersuchungen verliefen wie folgt : man entnahm eine Urinprobe, setzte dieser eine wäßrige Albuminlösung in einem Verhältnis von 9:1, 7:1 oder 5:1 zu, trug die erhaltene Mischung in Form eines Tropfens auf einen Objektträger auf und trocknete bei Raumtemperatur innerhalb von 24 Stunden. Danach prüfte man auf das Vorhandensein von Kristallen in der Randzone der getrockneten Probe. Die Prüfergebnisse sind in Tabellen 1 und 2 zusammengestellt.

**Tabelle 1**

| Klinischer und Laborbefund steinausscheidender Kranker | | | | | | |
|---|---|---|---|---|---|---|
| Lfd. Nr. | Probanden | Urin/wäßrige Albuminlösung-Verhältnis | | | Steinbildungsaktivitätsgrad | Entdeckungszeiten bei späteren klinischen Untersuchungen (in Monaten) |
| | | 9:1 | 7:1 | 5:1 | | |
| 1 | 2 | 3 | 4 | 5 | 6 | 7 |
| 1. | Kranker B. | + | - | - | schwach | 27 |
| 2. | Kranker L. | + | - | - | schwach | 24 |
| 3. | Kranker Ja. | + | - | - | schwach | 29 |
| 4. | Kranker Sch. | +++ | ++ | - | mäßig | 7 |
| 5. | Kranker M. | +++ | ++ | - | mäßig | 8 |
| 6. | Kranker R. | +++ | ++ | - | mäßig | 8 |
| 7. | Kranker O. | +++ | ++ | - | mäßig | 7 |
| 8. | Kranker K. | +++ | ++ | - | mäßig | 9 |
| 9. | Kranker S. | +++ | ++ | - | mäßig | 8 |
| 10. | Kranker U. | +++ | ++ | - | mäßig | 7 |
| 11. | Kranker A. | +++ | +++ | +++ | ausgeprägt | 3 |
| 12. | Kranker D. | +++ | +++ | +++ | ausgeprägt | 2 |
| Anmerkung : (+) Vorhandensein einzelner Kristalle in der Randzone der Probe (++) Kristallkonglomerate (+++) durchgehende Kristallisation der Randzone | | | | | | |

**Tabelle 2**

| Klinischer und Laborbefund praktisch gesunder Leute in der vorklinischen Stufe der Urolithiasis | | | | | | |
|---|---|---|---|---|---|---|
| Lfd. Nr. | Zahl d.untersuchten Leute | Urin/wäßrige Albuminlösung-Verhältnis | | | Steinbildungsaktivitätsgrad | Entdeckungszeiten b.späteren klinischen Untersuchungen (in Monaten) |
| | | 9:1 | 7:1 | 5:1 | | |
| 1 | 2 | 3 | 4 | 5 | 6 | 7 |
| 1. | 61 | + | - | - | schwach | 24-30 |
| 2. | 49 | +++ | ++ | - | mäßig | 7-8 |
| 3. | 18 | +++ | +++ | +++ | ausgeprägt | 2-3 |
| Anmerkung : (+) Vorhandensein einzelner Kristalle in der Randzone der Probe (++) Kristallkonglomerate (+++) durchgehende Kristallisation der Randzone | | | | | | |

Wie den in Tabellen 1 und 2 zusammengestellten Ergebnissen zu entnehmen ist, wurde eine 100%ige Übereinstimmung des Laborbefundes mit dem klinischen- auf Steinbildung deutenden - Befund festgestellt. Bei einer Gruppe steinausscheidender Kranker mit ausgeprägtem Steinbildungsaktivitätsgrad (volle Kristallisation der Randzone) beobachtete man die Steinbildung in einem Zeitabstand von 2 bis 3 Monaten, bei einer anderen Gruppe mit teilweiser Kristallisation der Randzone (Kristallkonglomerate ) einen gemäßigten Steinbildungsgrad nach Ablauf von 7 bis 8 Monaten und bei einer dritten Gruppe mit Einzelkristallen in der Randzone (schwachem Steinbildungsgrad) die Steinbildung nach Ablauf von 2 bis 2,5 Jahren. Das Gleiche trifft auch für den klinischen und den Laborbefund der proktisch gesunden Leute in ihrer vorklinischen Urolithiasisstufe (Tabelle 2) zu. Demzufolge läßt das angemeldete Verfahren auf die Intensität des Steinbildungsvorganges (den Aktivitätsgrad) und die Zeiten der Steinbildung schließen.

Parallel zur Bestimmung des Steinbildungsaktivitätsgrades von 13 an Urolithiasis leidenden Leuten (mit festgestellem ausgeprägtem Steinbildungsgrad) wurden Untersuchungen der Zusammensetzung steinbildender Salze realisiert. Unter den 13 Kranken waren 6 steinausscheidende Kranke und 7 einer Lithotripsie ausgesetzte Kranke. Ausgehend von einer auf einem Objektträger getrockneten Probe, welche durch eine volle Kristallisation der Randzone gekennzeichnet war, (das zeugt von einem ausgeprägten Steinbildungsgrad), wurde mit Hilfe einer Röntgenstrukturanalyse und einer Röntgenspektralmikroanalyse die Phasen- oder Elementzusammensetzung dieser kristallinen Bildungen bestimmt. Die erhaltenen Ergebnisse für die Elementzusammensetzung (Phasenzusammensetzung) der kristallinen Bildungen in der Randzone der Proben verglich man mit dem Analysenbefund der später ausgeschiedenen Steine.

Die Prüfergebnisse sind in Tabelle 3 zusammengestellt.

**Tabelle 3**

| Ergebnisse einer Vergleichsanalyse der Salzzusammensetzung kristalliner Bildungen in der Randzone einer Urinprobe und der ausgeschiedenen Urinsteine | | | | |
|---|---|---|---|---|
| Lfd. Nr. | Probanden Diagnose | | Zusammensetzung (Element-, Phasenzusammenstz.) der Salze kristal. Bildungen in d.Randzone d.Probe | Zusammensetzung ausgeschied. Urinsteine (vorherrsch Salzanteil in d. oberflächennahen Schicht |
| 1 | 2 | 3 | 4 | 5 |
| 1. | Kranker K. | Harnsteinleiden | Harnsäure* | Urate |
| 2. | Kranker P. | Harnsteinleiden | Harnsäure | Urate |
| 3. | Kranker Ch. | Harnsteinleiden | Harnsäure Saueres Ammoniumurat | Urate |
| 4. | Kranker L. | Harnsteinleiden | Harnsäure* | Urate |
| 5. | Kranker R. | Harnsteinleiden Sekundärpyelonephritis | Kalzium, Phosphor,Magnesium** | Phosphate |
| 6. | Kranker A. | Harnsteinleiden Sekundärpyelonephritis | Kalzium, Phosphor, Magnesium | Phosphate |
| 7. | Kranker N. | Harnsteinleiden Sekundärpyelonephritis | Phosphor, Kalzium, Zink** | Phosphate |
| 8. | Kranker W. | Harnsteinleiden Sekundärpyelonephritis | Phosphor, Kalzium** | Phosphate |
| 9. | Kranker A. | Harnsteinleiden | Phosphor, Kalzium, Magnesium** | Phosphate |
| 10. | Kranker I. | Harnsteinleiden | Phosphor, Kalzium** | Phosphate |
| 11. | Kranker G. | Harnsteinleiden Sekundärpyelonephritis | Kalzium** | Oxalate |
| 12. | Kranker T. | Harnsteinleiden Sekundärpyelonephritis | Kalzium | Oxalate |
| 13. | Kranker A. | Harnsteinleiden Sekundärpyelonephritis | Kalzium | Oxalate |

| | | | | |
|---|---|---|---|---|
| * Ergebnisse einer Röntgenstrukturanalyse | | | | |
| ** Ergebnisse einer Röntgenspektralanalyse | | | | |

Wie sich aus den Ergebnissen der Tab. 3 ableitet, stimmen die Analysenangaben zur Zusammensetzung der kristallinen Bildungen in der Randzone mit den Prüfergebnissen der Harnsteinzusammensetzung überein.

Somit erlaubt das anmeldungsgemäße Verfahren, die Steinsalzzusammensetzung in vorklinischen Entwicklungsstufen der Urolithiasis vorherzusagen oder zu bestimmen, wodurch einer Konkrementbildung vorgebeugt werden kann.

Um die vorliegende Erfindung näher zu erläutern, werden im folgenden konkrete Ausführungsbeispiele der Erfindung angeführt.

### Beispiel 1

Der Patient B. in einem Alter von 50 Jahren wurde durch ein klinisches Laboratorium innerhalb von 2 Jahren beobachtet. Unter die dynamische Beobachtung wurde er nach der Entfernung eines Steines aus dem Organismus gestellt. Jede Woche erfolgte die Harnschau unter Verwendung des angemeldeten Verfahrens. Man ging dabei so vor, daß einer Urinprobe eine wäßrige Eiweißlösung bei einem Verhältnis von 9:1, 7:1 oder 5:1 entsprechend zugesetzt wurde. Tropfen des so hergestellten Gemisches wurden auf einen Objektträger aufgebracht und bei Raumtemperatur innerhalb von 24 Stunden getrocknet. Anschließend bestimmte man die Art der Salzkristallisation in der Randzone der Probe. Innerhalb von vier Monaten war ein ausgeprägter Steinbildungsaktivitätsgrad (eine vollständige Kristallisation der Randzone der Probe) feststellbar. Parallel dazu wurde mit Hilfe einer Spektralmikroanalyse die Elementzusammensetzung der kristallinen Bildungen in der Randzone identifiziert. Um eine Vergleichsanalyse anstellen zu können, bestimmte man zugleich die Elementzusammensetzung der Mittelzone der Probe. Eine beachtliche Überschreitung des Gehaltes einzelner Elemente in der Randzone gegenüber der Mittelzone bildet die Grundlage für steinbildende Salze.

Die Ergebnisse aus der Bestimmung der Zusammensetzung steinbildender Salze wurden durch eine Analyse der Steinzusammensetzung nach der Entfernung dieses Steines aus dem Organismus nachgewiesen. Die Untersuchungsergebnisse sind in Tabelle 4 zusammengestellt.

**Tabelle 4**

| Angaben zu einer Vergleichsanalyse der Elementzusammensetzung in der Mittel- und Randzone einer Urinprobe und eines entfernten Harnsteines | | | | | | | | | | |
|---|---|---|---|---|---|---|---|---|---|---|
| Lfd. Nr. | Material | Elemente, % | | | | | | | | |
| | | Na | Mg | P | S | Cl | K | Ca | Zn | Si |
| 1 | 2 | 3 | 4 | 5 | 6 | 7 | 8 | 9 | 10 | 11 |
| 1. | Urinprobe (Mittelzone) | 27,4 | 1,1 | 3,4 | 1,1 | 62,6 | 3,3 | - | - | - |
| 2. | Urinprobe (Randzone) | 17,3 | 0,9 | 14,6 | 9,5 | 39,6 | 11,6 | 6,1 | - | - |
| 3. | Entfernter Harnstein | - | 3,3 | 30,3 | - | - | - | 64,2 | - | - |

Wie den Angaben der Tabelle 4 zu entnehmen ist, dienen als steinbildende Salzelemente Ca und P, was eben durch die Steinzusammensetzung (nach der Steinentfernung aus dem Organismus) nachgewiesen wurde.

Nach der Steinentfernung aus dem Organismus wurde dem Patienten eine medikamentöse Behandlung verordnet und die wöchentliche Urinschau durch das anmeldungsgemäße Verfahren innerhalb von 2 Jahren weiter getätigt. Die Ergebnisse der dynamischen Beobachtung (der regelmäßigen Bestimmung des Steinbildungsaktivitätsgrades) dieses Patienten sind der Tabelle 5 entnehmbar.

**Tabelle 5**

| Ergebnisse der Beobachtung der Steinbildungsaktivität des Kranken B. in der Dynamik nach Behandlungsbeginn | | | | |
|---|---|---|---|---|
| Untersuchungsdauer | Kristallisationsindexe einer Urinprobe in d.Mittelzone bei einem Urin/wäßrige Albuminlösung-Verhältnis von: | | | Aktivitätsgrad d. Steinbildung |
| | 9:1 | 7:1 | 5:1 | |
| 1 | 2 | 3 | 4 | 5 |
| 1 Woche | ++ | ++ | - | Gemäßigt |
| 1 Monat | + | + | - | schwach |
| 2-4 Monate | - | - | - | kein Prozeß |
| 5-7 Monate | ++ | + | - | gemäßigt |
| 8 Monate | + | - | - | schwach |
| 9-13 Monate | - | - | - | kein Prozeß |
| 14 Monate | + | - | - | schwach |
| 15-20 Monate | + | - | - | schwach |
| Anmerkung : + : Vorhandensein einzelner Kristalle ++ : Kristallkonglomerate - : keine Kristalle | | | | |

### Beispiel 2

Die Bestimmung des Steinbildungsaktivitätsgrades in einer Urinprobe des Kranken X. wurde in ähnlicher Weise wie im Beispiel 1 beschrieben durchgeführt. Es wurde ein ausgeprägter Steinbildungsaktivitätsgrad (eine vollständige Kristallisation in der Randzone einer Urinprobe) festgestellt. Mit Hilfe einer Röntgenspektralmikroanalyse ermittelte man die Elementzusammensetzung kristalliner Bildungen in der Randzone. Man stellte eine Vergleichsanalyse der Elementzusammensetzung der getrockneten Urinprobe in der Rand- und Mittelzone an. Die bei der Ermittlung der steinbildenden Salzzusammensetzung erreichten Ergebnisse wurden durch die Analyse der Zusammensetzung des aus dem Organismus entfernten Steines nachgewiesen. Die Prüfungsergebnisse sind in der Tabelle 6 zusammengestellt.

**Tabelle 6**

| Bei der Vergleichsanalyse der Elementzusammensetzung einer Urinprobe in der Mittel- und Randzone und eines entfernten Harnsteines erreichte Ergebnisse | | | | | | | | | | |
|---|---|---|---|---|---|---|---|---|---|---|
| Ldf. Nr. | Material | Elemente in % | | | | | | | | |
| | | Na | Mg | P | S | Cl | K | Ca | Zn | Si |
| 1. | Urinprobe (Mittelzone) | 26,2 | 2,0 | 15,1 | 8,9 | 30,5 | 13,0 | 2,6 | - | - |
| 2. | Urinprobe (Randzone) | 27,6 | 2,1 | 19,6 | 12,3 | 26,0 | 4,8 | 7,6 | - | - |
| 3. | Entfernter Harnstein | 1,5 | - | 4,1 | 0,5 | - | - | 93,0 | - | - |

Wie der Tabelle 6 entnehmbar ist, fungiert als Element der steinbildenden Salze vornehmlich Ca, was auch durch die Steinzusammensetzung bestätigt wird : Ca = 93 %.

### Beispiel 3

Die Bestimmung des Steinbildungsaktivitätsgrades in einer Urinprobe des Kranken K. erfolgte in ähnlicher Weise, wie das im Beispiel 1 beschrieben wurde. Man hat dabei einen ausgeprägten Aktivitätsgrad der Steinbildung (eine vollständige Kristallisation der Urinprobe in der Randzone) festgestellt. Mit Hilfe einer Röntgenspektralmikroanalyse ermittelte man die Elementzusammensetzung der kristallinen Bildungen in der Randzone. Man realisierte eine Vergleichsanalyse für die Elementzusammensetzung der getrockneten Urinprobe in der Rand- und Mittelzone. Die bei der Bestimmung der Zusammensetzung der steinbildenden Salze erreichten Ergebnisse wurden durch die Zusammensetzung des Steines nach dessen Entfernung aus dem Organismus bestätigt. Die Prüfungsergebnisse sind in Tabelle 7 zusammengefaßt.

**Tabelle 7**

| Ergebnisse für eine Vergleichsanalyse der Elementzusammensetzung einer Urinprobe in der Mittel- und Randzone und eines entfernten Urinsteines | | | | | | | | | | |
|---|---|---|---|---|---|---|---|---|---|---|
| Ldf. Nr. | Material | Elemente in % | | | | | | | | |
| | | Na | Mg | P | S | Cl | K | Ca | Zn | Si |
| 1. | Urinprobe (Mittelzone) | 17,2 | - | 15,8 | 12,2 | 31,1 | 19,7 | 2,6 | - | - |
| 2. | Urinprobe (Randzone) | 12,7 | 3,9 | 23,3 | 9,2 | 22,4 | 12,5 | 5,5 | - | - |
| 3. | Entfernter Harnstein | - | 14,0 | 39,3 | - | - | - | 44,7 | - | - |

Wie sich aus den Angaben der Tabelle 7 ableitet, dienen als Elemente der steinbildenden Salze vorwiegend Ca, P und Mg, was durch die Steinzusammensetzung nachgewiesen wurde : Ca 44,7%; P 39,3 % und Mg 14,0 %.

### Beispiel 4

Die Ermittlung des Steinbildungsaktivitätsgrades in einer Urinprobe des Kranken C. wurde ähnlich wie im Beispiel 1 beschrieben vorgenommen. Man stellte einen ausgeprägten Steinbildungsaktivitätsgrad (eine volle Kristallisation der Randzone) fest. Bei Anwendung einer Röntgenspektralmikroanalyse ermittelte man die Elementzusammensetzung kristalliner Bildungen in der Randzone. Es wurde eine Vergleichsanalyse für die Elementzusammensetzung einer getrockneten Urinprobe in der Rand- und Mittelzone angestellt. Nach Feststellen eines ausgeprägten Steinbildungsaktivitätsgrades und der Zusammensetzung der steinbildenden Harnsalze machte der Kranke eine individuelle Therapie durch. Man hat eine wiederholte Analyse einer Urinprobe angestellt. Die Prüfungsergebnisse sind in Tabelle 8 dargestellt.

**Tabelle 8**

| Bei einer Vergleichsanalyse der Elementzusammensetzung einer Urinprobe in der Mittel- und Randzone und eines entfernten Harnsteines erzielte Ergebnisse (in %) | | | | | | | | | | | |
|---|---|---|---|---|---|---|---|---|---|---|---|
| Untersuchungszeit | Steinbildungsaktivitätsgrad | Material | Elemente | | | | | | | | |
| | | | Na | Mg | P | S | Cl | K | Ca | Zn | Si |
| Vor d.Behandlung | Ausgeprägt | Urinprobe (Mittelzone) | 25,2 | 1,5 | 7,1 | 7,8 | 37,1 | 16,4 | 1,7 | 0,2 | - |
| | | Urinprobe (Randzone) | 14,1 | 0,2 | 14,6 | 2,7 | 17,0 | 8,4 | 4,7 | 0,2 | - |
| Nach d.Behandlung | kein Prozeß | Urinprobe (Mittelzone) | 33,4 | 1,0 | 10,6 | 10,1 | 35,9 | 7,1 | 1,4 | 1,3 | - |
| | | Urinprobe (Randzone) | 34,6 | - | 10,6 | 8,3 | 35,9 | 6,4 | 0,4 | 1,1 | - |

Wie aus der Tabelle 8 ersichtlich ist, dienen als Elemente der steinbildenden Salze (vor der Behandlung) Ca und P ; nach der Behandlung war kein Steinbildungsprozeß zu verzeichnen.

### Gewerbliche Anwendbarkeit

Das anmeldungsgemäße Verfahren kann bei klinischen und wissenschaftlichen Laboruntersuchungen zwecks Analyse des Steinbildungsaktivitätsgrades Eingang finden ; es ist weiter bei Untersuchungen des Steinbildungsvorganges in der Dynamik sowie bei der Bestimmung der Salzzusammensetzung des Harnsteines in frühen Urolithiasisstufen zur Vorbeuge der Harnsteinbildung und zur Festlegung einer individuellen Therapie bei der Urolithiasis anwendbar.

## Patentansprüche

1. Verfahren zur Bestimmung des Steinbildungsaktivitätsgrades und der Zusammensetzung steinbildender Urinsalze bei einer Urolithiasis, durch Zusatz einer wäßrigen Eiweißlösung zu einer Urinprobe, Aufbringung der erhaltenen Mischung in Form eines Tropfens auf eine ebene Oberfläche, Trocknung innerhalb von 24 Stunden und Ermittlung der Art der Salzkristallisation in der Randzone der Probe,
dadurch gekennzeichnet, daß der Urinprobe die Eiweißlösung im Verhältnis 9:1, 7:1 oder 5:1 zugesetzt wird und daß bei Vorhandensein einzelner Kristalle, deren Konglomerate oder bei vollständiger Kristallisation dieser Zone über einen schwachen, gemäßigten oder ausgeprägten Steinbildungsaktivitätsgrad geschlußfolgert und die Zusammensetzung dieser kristallinen Bildungen unter paralleler Bestimmung der Salzzusammensetzung in der Mittelzone der Probe und bei späterer Durchführung einer Vergleichsanalyse dieser Zusammensetzungen zwecks Bestimmung der steinbildenden Urinsalze ermittelt wird.

2. Verfahren nach Anspruch 1,
dadurch gekennzeichnet, daß als wäßrige Eiweißlösung eine 8 - 12%ige wäßrige Albuminlösung zum Einsatz gelangt.

3. Verfahren nach Anspruch 1 oder 2,
dadurch gekennzeichnet, daß das Trocknen eines auf die ebene Oberfläche aufgetragenen Tropfens der Mischung bei Raumtemperatur realisiert wird.

4. Verfahren nach einem der Ansprüche 1 bis 3,
dadurch gekennzeichnet, daß zur Bestimmung der Elementzusammensetzung kristalliner Bildungen eine Röntgenspektralmikroanalyse durchgeführt wird.

## Claims

1. A method of determining the degree of stone formation activity and the composition of stone-forming urine salts in the case of urolithiasis through adding an aqueous protein solution to a urine specimen, applying the obtained mixture to a plane surface in the form of a drop, drying within 24 hours and determining the type of salt crystallisation in the edge zone of the specimen, characterised in that the protein solution is added to the urine specimen in the ratio 9:1, 7:1 or 5:1, and in that with the presence of individual crystals, their conglomerates or with total crystallisation of this zone, conclusions are drawn regarding a low, moderate or marked degree of stone formation activity, and the composition of these crystalline formations is determined accompanied by parallel determination of the salt composition in the central zone of the specimen and with a comparative analysis of these compositions being carried out subsequently for the purpose of determining the stone-forming urine salts.

2. A method in accordance with Claim 1, characterised in that a 8 - 12% aqueous albumin solution is used as an aqueous protein solution.

3. A method in accordance with Claim 1 or 2, characterised in that drying of a mixture drop applied to the plane surface takes place at room temperature.

4. A method in accordance with any one of Claims 1 to 3, characterised in that an X-ray spectrometric microanalysis is carried out to determine the element composition of crystalline formations.

## Revendications

1. Procédé de détermination du degré d'activité de formation de calculs et de la composition de sels urinaires formant des calculs lors d'une lithiase urinaire. par addition d'une solution aqueuse d'albumine à un échantillon d'urine, application du mélange obtenu sous forme d'une goutte sur un support plan, séchage en l'espace de 24 heures et caractérisation du type de cristallisation saline dans les zones en bordure de l'échantillon, caractérisé en ce que l'on ajoute, à l'échantillon d'urine, la solution protéique en un rapport de 9:1, 7:1 ou 5:1 et que l'on déduit de la présence de cristaux isolés, de leurs agglomérats, ou de cristallisation complète de cette zone, une activité faible, moyenne ou prononcée de formation de calculs et en ce que l'on détermine la composition de ces formations cristallines en déterminant parallèlement la composition saline de la zone centrale de l'échantillon et en exécutant ensuite une analyse de comparaison de ces compositions afin de déterminer les sels d'urines formant des calculs.

2. Procédé selon la revendication 1, caractérisé en ce que l'on met en oeuvre comme solution aqueuse d'albumine une solution d'albumine aqueuse à 8-12%.

3. Procédé selon la revendication 1 ou 2, caractérisé en ce que le séchage de la goutte du mélange appliquée sur une surface plane est réalisé à température ambiante.

4. Procédé selon la revendication 1 à 3, caractérisé en ce que l'on réalise, pour la détermination de la composition élémentaire des compositions cristallines, une microanalyse spectrale aux rayons-X.
